Europäisches Patentamt

European Patent Office

Office européen des brevets

(19)

(11) Publication number: **0 050 457**
**B1**

(12) **EUROPEAN PATENT SPECIFICATION**

(45) Date of publication of patent specification: **27.12.85**

(21) Application number: **81304706.5**

(22) Date of filing: **09.10.81**

(51) Int. Cl.⁴: **C 09 J 3/14, C 09 K 9/02, A 61 K 49/04**

(54) **Radiopaque cyanoacrylate compositions.**

(30) Priority: **20.10.80 US 198466**
**26.05.81 US 267400**

(43) Date of publication of application:
**28.04.82 Bulletin 82/17**

(45) Publication of the grant of the patent:
**27.12.85 Bulletin 85/52**

(84) Designated Contracting States:
**CH DE FR GB IT LI NL SE**

(56) References cited:
**FR-A-2 348 699**
**FR-A-2 352 544**

**Patents Abstracts of Japan Vol. 5, No. 54, 15 April 1981**

The file contains technical information submitted after the application was filed and not included in this specification

(73) Proprietor: **BioNexus, Inc.**
**5257 North Boulevard**
**Raleigh North Carolina 27604 (US)**

(72) Inventor: **Krall, Robert E.**
**P.O. BOx 319**
**Georgetown Grand Cayman (KY)**

(74) Representative: **Skailes, Humphrey John et al**
**Frank B. Dehn & Co. Imperial House 15-19 Kingsway**
**London WC2B 6UZ (GB)**

(56) References cited:

**Chemical Abstracts vol. 94, no. 24, 15 June 1981 Columbus, Ohio, USA W. SMIGIELSKI et al. "Urogranoic acid as a radiopaque additive to the cyanoacrylic adhesive in transcatheter obliteration of renal arteries" page 353, column 1, abstract no. 197519w**

**Chemical Abstracts vol. 84, no. 20 17 May 1976 Columbus, Ohio, USA W. KUBIAK "Acryl substance for prosthetics and implantations" page 361, column 1, abstract no. 140763k**

Courier Press, Leamington Spa, England.

## Description

Methyl cyanoacrylate (MCA) and other cyanoacrylates are well known chemical products which have found wide use in industry and in the medical field for a variety of purposes, e.g. as a tissue adhesive.

Thus, the ability of the alkyl 2-cyanoacrylate to polymerize rapidly at room temperature in the absence of a solvent or added catalyst has resulted in the use of this class of monomers for surgical application. 2-cyanoacrylate monomers have been used as physiological adhesives in bronchial closure, the anastomosis of small arteries, intestinal anastomosis and cutaneous lacerations. Methyl cyanoacrylate has found application in the field of permanent sterilization of human females. Sterilization of the female is accomplished by the introduction of small quantities of MCA into the fallopian tubes wherein contact with body moisture polymerizes the MCA and blocks the fallopian tube. With passage of time, fiberous tissue growth replaces the MCA and permanent sterilization results. This latter use and procedure is described in U.S. Patent No. 3,822,702 and 3,948,259.

For some of the medical applications of the cyanoacrylates, it is desirable that there be some means for inspecting the result of the surgical operation without the need for surgically reopening the patient. For instance, in the anastomosis of the arteries or intestines, it is desirable to have some means for non-intrusively inspecting the joined tubes in order to verify that a proper weld has taken place without blockage of the tube. Conversely, where MCA is used to sterilize females it is desirable to inspect the polymer plug to insure that tube blockage has been successfully accomplished.

Cyanoacrylates are also used industrially as adhesives, e.g. for wood, metal, plastic or ceramic parts, and in these applications it is also desirable to be able to inspect the product, particularly for example in the case of hidden seams, welds or plugs.

If the cyanoacrylate ester is made radiopaque, the desired inspection may be accomplished by x-ray of the affected area. However, the polymerization properties which make cyanoacrylate esters useful in medical applications have heretofore prevented formulation of radiopaque compositions. The highly unstable cyanoacrylate esters polymerize under many conditions including exposure to even trace amounts of moisture, oxygen, heat, high energy radiation and active organic sites to note a few. As a result, many of the known radiopaque materials commonly used in other medical applications cannot be incorporated into the monomer as a radiopaque additive for cyanoacrylate esters. Most radiopaque materials reduce the stability of the esters and in some cases even initiate polymerization thereof. In particular, it has been found that organic iodo compounds commercially available as x-ray screening agents are frequently available as sodium salts of a carboxylic acid or as compounds containing primary or secondary amino groups. These materials are not stable to cyanoacrylates and cause polymerization thereof.

The present invention discloses a group of compounds which may be added to methyl cyanoacrylate and other cyanoacrylate esters in order to provide radiopaque compositions useful in medical and industrial applications where it is desired to inspect an adhesive weld or a polymer plug by x-ray techniques. The compositions generally comprise an alphacyanoacrylate ester and a radiopaque additive stable to such ester, the additive being triiodophenol or iodoform or a mixture thereof. The additives of the present invention do not cause short term rejection of the cyanoacrylate compositions of the present invention, do not otherwise cause the cyanoacrylate composition to become significantly toxic under conditions of use and that the additive will eventually be eliminated from the body. The compositions thus remain physiologically acceptable.

2,4,6-Triiodophenol is also useful as an anionic inhibitor and its use in the compositions of the present invention is especially advantageous because such use permits the elimination of the other inhibitors. This simplifies the complexity of the cyanoacrylate system and, in turn, simplifies the study of the biodegradation of the polymer system and reduces risk of unexpected side effects.

Additional inhibitors may be used to the compositions as required. It is known, for instance, to use $SO_2$ and antioxidants such as hydroquinone, butylated hydroxytoluene, butylated hydroxyanisole and hydroquinone monomethyl ether as cyanoacrylate inhibitors. For female sterilization applications, compositions utilizing a combination of $SO_2$, antioxidants and carboxylic acids such as acetic acid or benzoic acid may be used. Iodoform does not act as a anionic inhibitor or as a polymerization initiator.

While it is possible to use the materials disclosed herein as suspensions in cyanoacrylate esters, it is much preferred that the additive be completely dissolved in the ester. Complete solution assures accuracy and reproducibility of the additive concentration. Additionally, in some cases, particulate matter in the composition may reduce shelf life.

The radiopaque additive is generally present in an amount sufficient to provide 4—6 mole percent iodine.

It has been found that in some deep body work it is necessary that iodine have a concentration greater than 5 mole percent. In particular, in female sterilization applications, when attempting to locate the image of a fallopian tube polymer plug against the substantially opaque background of the pelvis, it has been found that a concentration of iodine atoms of about 7 mole percent is desirable in order to obtain a recognizable image.

An especially useful composition of this type is a heated equimolar mixture of triiodophenol and iodoform. Iodoform efficiently adds iodine to the cyanoacrylate compositions but does not remain in solution at levels adequate for female sterilization work. Triiodophenol has a relatively high iodine content along with an inhibition function. Also, both compounds have been well studied from a toxicological standpoint and are known to be relatively non-toxic.

### Example

A mixture of sterile redistilled MCA is prepared containing 1.17 mole percent of iodoform and 1.17 mole percent triiodophenol. The composition also contains 250—750 ppm (mole basis) $SO_2$ which is added as a stabilizer during the redistillation and sterilization step. About 250 ppm hydroquinone is added to decrease light sensitivity. The mixture is heated with stirring to 80°C for an hour in the dark (a low intensity, dark room type red light may be used). The resulting composition, containing 7 mole percent iodine atoms, may be stored for extended periods in aluminum foil or other containers which are opaque to visible to ultraviolet light. Upon exposure to light, e.g., just prior to use, the composition is stable for two to three hours. When used as a female sterilizing agent the polymer plug formed by this composition in the fallopian tube is distinguishable over the pelvic background and an x-ray image.

The fact that this composition is light sensitive, together with the need for heating to completely dissolve the additive materials, suggests that the composition involves a charge transfer complex rather than a single solution of two additives in MCA.

### Claims

1. A radiopaque polymerizable cyanoacrylate composition comprising a mixture of an ester of 2-cyanoacrylic acid and a radiopaque additive which is stable to the ester and is triiodophenol and/or iodoform.

2. A composition as claimed in claim 1 wherein the radiopaque additive is present in an amount sufficient to provide an iodine concentration of 4—6 mole percent iodine atoms.

3. A composition as claimed in claim 1 or claim 2 wherein the cyanoacrylate ester is methyl-2-cyanoacrylate.

4. A composition as claimed in any preceding claim wherein the radiopaque additives are triiodophenol and iodoform.

5. A composition as claimed in claim 4 wherein the triiodophenol and iodoform are present in approximately equal molar amounts.

6. A radiopaque polymerizable composition as claimed in claim 1 comprising a solution in methyl-2-cyano
acrylate of about 1.17 mole percent triiodophenol and about 1.17 mole percent iodoform.

7. A composition as claimed in claim 6 further containing about 250 mole ppm of hydroquinone and sulfur dioxide in a range of 250—750 mole ppm.

### Patentansprüche

1. Radioopake polymerisierbare Cyanoacrylat-Zusammensetzung, gekennzeichnet durch eine Mischung aus einem Ester der 2-Cyanoacrylsäure und einem radioopaken Additiv, welches gegenüber den Ester stabil und Trijodophenol und/oder Jodoform ist.

2. Zusammensetzung nach Anspruch 1, dadurch gekennzeichnet, daß das radioopake Additiv in einer ausreichenden Menge vorhanden ist, um eine Jodkonzentration von 4—6 Mol-% Jodatomen vorzusehen.

3. Zusammensetzung nach Anspruch 1 oder 2, dadurch gekennzeichnet, daß der Cyanoacrylatester Methyl-2-Cyanoacrylat ist.

4. Zusammensetzung nach jedem der vorhergehenden Ansprüche, dadurch gekennzeichnet, daß die radioopaken Additive Trijodophenol und Jodoform sind.

5. Zusammensetzung nach Anspruch 4, dadurch gekennzeichnet, daß das Trijodophenol und das Jodoform in annähernd gkeichen Molarmengen vorhanden sind.

6. Radioopake polymerisierbare Zusammensetzung nach Anspruch 1, gekennzeichnet durch eine Lösung von etwa 1.17 Mol-% Trijodophenol und etwa 1.17 Mol-% Jodoform in Methyl-2-cyanoacrylat.

7. Zusammensetzung nach Anspruch 6, ferner gekennzeichnet durch einen Gehalt von etwa 250 Mol/ppm Hydrochinon und Schwefeldioxid in einem Bereich von 250 bis 750 Mol/ppm.

### Revendications

1. Composition de cyanoacrylate polymérisable, opaque aux rayons X, caractérisée en ce qu'elle comprend un mélange d'un ester d'acide 2-cyanoacrylique et d'un additif opaque aux rayons X, qui est stable vis-à-vis de l'ester et qui est triiodophénol et/ou l'iodoforme.

2. Composition selon la revendication 1, caractérisée en ce que l'additif opaque aux rayons X est présent en quantité suffisante pour donner une concentration en iode de 4—6 moles % d'atomes d'iode.

3. Composition selon la revendication 1 ou 2, caractérisée en ce que l'ester cyanoacrylique est le 2-cyanoacrylate de méthyle.

4. Composition selon l'une quelconque des revendications précédentes, caractérisée en ce que les additifs opaques aux rayons X sont le triiodophenol et l'iodoforme.

5. Composition selon la revendication 4, caractérisée en ce que le triiodophenol et l'iodoforme sont présents em quantités molaires à peu près égales.

6. Composition polymérisable opaque aux rayons X selon la revendication 1, caractérisée en ce qu'elle comprend une solution dans le 2-cyanoacrylate de méthyle d'environ 1,17 moles % de triiodophénol et environ 1,17 moles % d'iodoforme.

7. Composition selon la revendication 6, caractérisée en ce qu'elle contient en outre environ 250 moles par million d'hydroquinone et du dioxyde soufre dans la gamme de 250—750 moles par million.